# EUROPEAN PATENT SPECIFICATION

(11) **EP 1 079 849 B1**
(45) Date of publication and mention of the grant of the patent: **02.01.2002**
(21) Application number: 99926330.4
(22) Date of filing: 19.05.1999
(51) Int. Cl.: A61K 38/17, C07K 14/47, A61K 39/395

(54) **USE OF HMG PROTEINS FOR THE PREPARATION OF MEDICAMENTS HAVING CYTOTOXIC ACTIVITY**
VERWENDUNG VON HMG PROTEINEN ZUR HERSTELLUNG VON ARZNEIMITTELN MIT ZYTOTOXISCHER WIRKUNG
UTILISATION DE PROTEINES HMG POUR L'ELABORATION DE MEDICAMENTS A ACTIVITE CYTOTOXIQUE

(30) Priority: 19.05.1998 IT MI981094
(43) Date of publication of application: 07.03.2001
(73) Proprietor: Istituto Di Ricerche " Giorgio Sisini", 20097 San Donato Milanese (IT)
(72) Inventor: BARTORELLI, Alberto, CH-3963 Crans sur Sierre (CH)
(74) Representative: Minoja, Fabrizio, Dr.
(86) International application number: EP9903431
(87) International publication number: WO9959609

(56) References cited:
- EP-A- 0 727 487
- EP-A- 0 791 601
- FR-A- 2 739 292
- PATENT ABSTRACTS OF JAPAN vol. 012, no. 011 (C-468), 13 January 1988 (1988-01-13) & JP 62 166897 A (TOYO SODA MFG CO LTD;OTHERS: 01), 23 July 1987 (1987-07-23)
- PASSALACQUA M ET AL: "Secretion and binding of HMG1 protein to the external surface of the membrane are required for murine erythroleukemia cell differentiation." FEBS LETTERS, (1997 JAN 6) 400 (3) 275-9. , XP002123136 cited in the application
- JAYARAMAN L ET AL: "High mobility group protein-1 (HMG-1) is a unique activator of p53." GENES AND DEVELOPMENT, (1998 FEB 15) 12 (4) 462-72. , XP002123137 cited in the application

## Description

The present invention refers to the use of HMG proteins for the preparation of medicaments having cytotoxic activity.

The invention further concerns peptides having amino acid sequence corresponding or homologous to the amino terminus region of HMG proteins.

The HMG proteins, also known as High Mobility Group proteins, belong to a family of highly conserved nucleoproteins associated to chromatin and involved in the regulation processes of gene expression.

Therapeutic uses of HMG proteins, (hereinafter HMG) have never been described. The most studied HMG proteins include the HMG-I and HMG-1 proteins.

A number of different Authors have recently reported more and more complex roles for HMG: for instance, Jayarama et al., Genes Dev.1998, 12(4):462-472 disclosed the role of HMG-I as activator of the p53 protein.

Zappavigna et al., The Embo J, 15(18), 16/9/1996, make the hypothesis that the HMG-I proteins are co-factors in the transcriptional activation mediated by products coded by the family of HOX genes.

Falciala et al., J. Cell. Biol. 1997, 137(1), 19-26 report that HMG-I is not associated in a stable way with somatic cells chromosomes, differently from Histone H1.

Passalacqua et al., FEBS Lett. 1997, 400(3),275-9 show that erythroleucemic murine cells accumulate HMG-I on the outer cell surface.

It has now been found that the HMG proteins or peptides derived from the sequences thereof induce in the serum of animals and humans a cytotoxic antibody response against tumor cells.

Said antibody response, mainly mediated by type G and M immunoglobulins ( IgG and IgM), involves significant therapeutic effects on neoplastic diseases, particularly in the treatment of carcinomas and adenocarcinomas.

It has also been found that the domain of HMG which is mainly responsible for the induction of the cytotoxic antibody response is situated at the N-terminus of the HMG proteins.

The invention provides therefore pharmaceutical compositions containing as the active ingredient a protein of the HMG group, a fragment thereof or a peptide with amino acid sequence corresponding to or derived from the HMG sequence.

Preferred HMG proteins include mammalian HMG-I and HMG-1 proteins.

Preferably, the fragment comprises the N-terminus sequence and the peptide has sequence corresponding or derived from the N-terminus sequence of HMG.

The term "HMG-I protein" or "HMG-1 proteins" refers to any non-histone chromosomial protein with an homology of at least 70%, preferably higher than 80%, and even more preferably higher than 90%, with the sequences of human HMG-I reported in the SWISS-PROT data-base under accession number P17096 or with the sequences of human HMG-1 reported in the SWISS-PROT data-base under accession number P09429 respectively.

The invention also refers to HMG mutants obtained by addition, deletions or conservative substitutions of amino acids . Said mutants may be obtained, for example, by site-directed mutagenesis methods.

The HMG which may be used according to the invention may be of different origin, preferably of mammalian origin, for instance of man, goat, mouse, pig, rabbit, rat.

The term" peptide having amino acid sequence corresponding to or derived from the sequence of HMG protein" refers to a peptide having from 10 to 40 amino acids, preferably from 10 to 30 amino acids and more preferably from 10 to 25 amino acids, with sequence having homology of at least 70%, and preferably higher than 80%, with the HMG sequences as defined above.

The peptide 1-19 of HMG-I is commercially available from Santa Cruz Biotechnology, Santa Cruz, CA, USA.

The invention refers also to the peptides different from that above reported, and to the mono- or polyclonal antibodies recognising said peptides and HMG. Said antibodies may used in therapy or in diagnostic and immunohistochemical methods.

The HMG may be prepared by conventional extractive or recombinant DNA methods. The genes coding for HMG are in fact mostly known and may be inserted into suitable expression vectors for prokaryotic or eukaryotic cells.

The peptides referred to in the invention may be prepared by conventional methods of peptide synthesis, both in solid and in homogeneous phase. The peptides of the invention may also be stabilised versus the metabolic degradation, e.g. versus proteases, by means of conventional methods. These methods rely on the use of non-natural D-amino acids, retro inverted bonds or substitutions on the terminal NH₂ or COOH groups or on other groups of the sequence amino acids.

The peptides referred to in the invention may also be inserted into the sequence of known proteins, for example albumin, to give chimeric proteins having desired stability, pharmacokinetic and carrier properties.

The amino acid sequences may also be changed by means of conservative amino acid substitutions, such as that disclosed in H. Neurath and R.L. Hill "The Proteins", Academic Press, N. Y., USA, 1979.

Alternatively, the peptides may be obtained by means of recombinant DNA methods. To this aim, suitable DNA sequences, obtained by synthesis or by PCR methods using suitable primers on HMG-I gene sequences, are inserted into expression vectors for prokaryotic or eukaryotic cells, according to known methods.

For the intended therapeutic uses, HMG, their fragments or peptides will be administered suitably formulated in pharmaceutical composition, for example as reported in "Remington's Pharmaceutical Sciences Handbook", Mack Publishing Company, New York, U.S.A..

The compositions referred to in the invention will contain an effective amount of the peptides (or the derivatives thereof and the chemeric proteins), for instance from 0.1 to 100 mg of peptide, and they will be administered preferably by the parenteral route, in particular by the subcutaneous or intramuscular routes. The daily amount will obviously depend on different factors, like severity of the disease, weight, sex and age of the patient, and it will be determined on the basis of the toxicological, pharmacokineticenic and parmacodynamic properties of each single peptide or derivative thereof.

Generally the peptide daily dosage will be comprised between 10 and 1500 µmol per Kg of body weight and the treatment will be maintained for a long time. Also other administration route can be used, for example the oral route using liposome formulations or other techniques known for the administration of peptides or proteins by the gastroenteric route, as described in WO93/25583.

It is also possible the sublingual administration, using granules or solutions to be dosed as drops. In such a case, the doses of HMG or peptides may also be remarkably lowered since it has been noticed that it is possible to obtain a marked efficacy through this administration route.

The invention is described in more detail in the following Example.

### EXAMPLE

50-60 days old female rats were treated with 30 mg of dimethylbenzanthracene (DMBA) by gastric probe, divided in three administrations separated by a seven days period. The tumors may be detected after 50-80 days.

Ninety rats were the randomly divided into three groups and treated once a week for three consecutive weeks with HMG-I ( 30 µg/rat), with the 1-19 peptide ( 50 µg/rat) ( Santa Cruz Biotechnology, Inc.) or with saline solution ( 0.5 ml/rat).

At the start of treatment, the rats weighed 170-200 g; every 7 days, the size of the tumors was measured by means of a caliber and the physical condition and the weight of the rats were checked.

At the end of the test, one week after the last injection, the animals were sacrificed by decapitation after ketamine hydrochloride anesthesia. The tumors were removed and subjected to microscopic examination. The obtained results are reported in the following Table.

HMG-I and the 1-19 peptide clearly slow down the tumor growth in comparison to the control .

The statistical analysis of the obtained data show a significant difference between control and treated groups, the latter giving comparable results.

Tumor growth in rats treated with HMG-I (30 µg/rat) and peptide 1-19 (50 µg /ml).

| Group | Mean area of the tumor at the following treatment day (cm²) | | | |
|---|---|---|---|---|
| | 0 | 7 | 14 | 21 |
| Controls | ND | 1,7±0,9 | 3,1±1,3 | 4,0±1,8 |
| HMG-I | ND | 0,7±0,2 | 1,1±0,7 | 1,2±0,7 |
| Peptide 1-19 | ND | 0,9±10,8 | 1,4±0,9 | 1,5±0,8 |

## Claims

1. Use of a protein of the HMG group, a fragment thereof or a peptide with amino acid sequence corresponding to or derived from the HMG sequence for the preparation of anti-tumor medicaments.

2. Use according to claim 1 wherein the HMG protein is selected from HMG-I or HMG-1 proteins.

3. Use according to claim 2, wherein the HMG-I or HMG-1 are of human origin or from mouse, rat, rabbit or pig.

4. Use according to claim 1 of a N-terminal fragment of HMG-I or HMG-1.

5. Use according to claim 1 of a peptide having from 10 to 40 amino acids with amino acid sequence homologous for at least 70% to the sequence of HMG group.

## Patentansprüche

1. Verwendung eines Proteins der HMG-Gruppe, eines Fragments davon oder eines Peptids mit einer Aminosäuresequenz, die der der HMG-Sequenz entspricht oder von dieser abgeleitet ist, zur Herstellung von Antitumor-Arzneimitteln.

2. Verwendung nach Anspruch 1, wobei das HMG-Protein aus HMG-I- oder HMG-1-Proteinen ausgewählt ist.

3. Verwendung nach Anspruch 2, wobei das HMG-I oder HMG-1 humanen Ursprungs ist oder aus Maus, Ratte, Kaninchen oder Schwein stammt.

4. Verwendung nach Anspruch 1 eines N-terminalen Fragments von HMG-I oder HMG-1.

5. Verwendung nach Anspruch 1 eines Peptids, das 10 bis 40 Aminosäuren mit einer Aminosäuresequenz, die zu mindestens 70% mit der Sequenz der HMG-Gruppe homolog ist, hat.

## Revendications

1. Utilisation d'une protéine du groupe HMG, d'un fragment de celle-ci ou d'un peptide avec une séquence d'acides aminés correspondant à ou dérivée de la séquence HMG pour la préparation de médicaments antitumoraux.

2. Utilisation selon la revendication 1, dans laquelle la protéine HMG est choisie parmi les protéines HMG-I ou HMG-1.

3. Utilisation selon la revendication 2, dans laquelle la HMG-I ou HMG-1 est d'origine humaine ou de souris, de rat, de lapin ou de cochon.

4. Utilisation selon la revendication 1 d'un fragment de l'extrémité N-terminale de HMG-I ou HMG-1.

5. Utilisation selon la revendication 1 d'un peptide ayant de 10 à 40 acides aminés avec une séquence d'acides aminés homologue pour au moins 70 % à la séquence du groupe HMG.
